(19) Europäisches Patentamt
European Patent Office
Office européen des brevets

(11) EP 3 887 334 B1

(12) FASCICULE DE BREVET EUROPEEN

(45) Date de publication et mention de la délivrance du brevet:
**09.04.2025 Bulletin 2025/15**

(21) Numéro de dépôt: **19805990.9**

(22) Date de dépôt: **25.11.2019**

(51) Classification Internationale des Brevets (IPC):
***C04B 28/02*** $^{(2006.01)}$

(52) Classification Coopérative des Brevets (CPC):
(C-Sets disponibles)
**C04B 28/02; C04B 40/0039;** C04B 2111/82
(Cont.)

(86) Numéro de dépôt international:
**PCT/EP2019/082427**

(87) Numéro de publication internationale:
**WO 2020/109231 (04.06.2020 Gazette 2020/23)**

# (54) MÉTHODE D'ANALYSE DE LA QUANTITÉ D'ARGILE DANS UN SABLE

VERFAHREN ZUR ANALYSE DER TONMENGE IN EINEM SAND

METHOD FOR ANALYSING THE QUANTITY OF CLAY IN A SAND

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2018 FR 1871917**

(43) Date de publication de la demande:
**06.10.2021 Bulletin 2021/40**

(73) Titulaire: **CHRYSO**
**92400 Courbevoie (FR)**

(72) Inventeurs:
- **DERLY, Christophe**
  **45480 IZY (FR)**
- **COLAS, Antoine**
  **92340 BOURG-LA-REINE (FR)**

(74) Mandataire: **Lavoix**
**2, place d'Estienne d'Orves**
**75441 Paris Cedex 09 (FR)**

(56) Documents cités:
**EP-A1- 1 015 398**
**WO-A1-2011/121230**
**WO-A2-2006/032786**
**FR-A1- 2 875 496**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 887 334 B1

(52) Classification Coopérative des Brevets (CPC):
(Cont.)

C-Sets
**C04B 28/02, C04B 14/10, C04B 40/0032, C04B 2103/32;**
**C04B 40/0039, C04B 14/10, C04B 24/32**

## Description

La présente invention concerne une méthode d'analyse de la quantité d'argile dans un sable. Cette méthode permet notamment d'anticiper la surconsommation en superplastifiant du fait de la présence des argiles et ainsi agir en conséquence.

La présence d'argiles sodiques, telles que la Montmorillonite, dans les sables ou dans les granulats peut affecter fortement l'ouvrabilité des compositions de liant hydraulique, notamment des compositions de béton. En effet, la structure en feuillets des argiles favorise l'absorption de l'eau et l'intercalation des éléments contenus dans les compositions de liant hydraulique, tels que par exemple les superplastifiants et notamment ceux porteurs de greffons poly(alkylène glycol). Ces phénomènes provoquent une augmentation de la viscosité de la pâte de liant hydraulique, et donc une perte d'ouvrabilité. Un surdosage en superplastifiant est alors nécessaire pour pallier sa consommation par les argiles et maintenir l'ouvrabilité désirée. Ce phénomène n'est pas observé pour les argiles calciques.

Le dosage de la quantité d'argile se fait, généralement, par l'essai au bleu de méthylène (*Norme NF EN 933-9).* Cependant, cette analyse n'est pas sélective des argiles posant potentiellement problème et de plus elle est sensible à la quantité de fine des sables.

Il y a donc un intérêt à proposer un protocole d'analyse pour la quantification du taux d'argiles consommatrices de superplastifiant dans les sables permettant notamment la prédiction d'un dosage en Agent Modificateur de l'Activité de l'Argile (AMAA).

Les documents EP 1 015 398, WO 2011/121230, WO 2006/032786 et FR 2 875 496 divulguent des procedes d'inertage d'argiles dans des sables destines a la preparation de compositions hydrauliques. Dans ces procedes le sable est mis en contact avec un produit inertant d'argiles.

Les publications Prajapati et al, 2005 (A. K. Prajapati et al.: « Azomesogens with methoxyethyl tail : Synthesis and characterization », PROCEEDINGS IF THE INDIAN ACADEMY OF SCIENCES. CHEMICAL SCIENCES, vol. 117, no. 3, mai 2005, pages 255-261, XP055602304) et Prajapati et al, 2004 (A. K. Prajapati et al.: « Azomesogens containing an ethoxyethyl terminal chain : synthesis and characterization », LIQUID CRYSTALS, TAYLOR & FRANCIS, GB, vol. 31, no. 6, juin 2004, pages 889-894, XP001195475) divulguent des dérivés de type azobenzène.

La publication Peng et al, 2014 (Shuhua Peng et al.: « Azobenzene moiety variation directing self-assembly and photoresponsive behavior of azo-surfactants », JOURNAL OF MATERIALS CHEMISTRY C, vol. 2, no. 39, 21 mai 2014, pages 8303-8312, XP055602312) divulgue un composé B de type azobenzène.

La demande JPH101628 A divulgue également un composé de type azobenzène.

Un objectif de la présente invention est donc de fournir un procédé d'analyse pour la quantification du taux d'argiles consommatrices de superplastifiant.

Un autre objectif de la présente invention est de fournir un tel procédé permettant la prédiction d'un dosage en AMAA.

Un autre objectif encore de la présente invention est de fournir un tel procédé qui soit simple et puisse être utilisable sur le terrain.

D'autres objectifs encore apparaîtront à la lecture de la description de l'invention qui suit.

Tous ces objectifs sont remplis par la présente invention qui concerne l'utilisation de composé de formule (I) pour analyser et déterminer la quantité d'argile dans un sable, notamment pour déterminer par colorimétrie la quantité d'argile dans un sable,

$$R^1\text{-}(OA)_n\text{-}XR^2 \qquad \text{(I)}$$

dans laquelle

$R^1$ représente un groupe alkyle en C1 à C4, linéaire ou ramifié, ou un composé coloré ; $R^2$ représente un composé coloré;
A, chacun identique ou différent, représente indépendamment un groupe $-CH_2-CH_2-$ ou un groupe $-CH(CH_3)-CH_2-$ ;
n représente un entier compris entre 1 et 500, de préférence compris entre 4 et 250 X est O ou NH.

Dans le cadre de la présente invention, on entend par composé coloré, tout type de composé présentant une adsorption des rayonnements dont les longueurs d'onde appartiennent au domaine du visible. Il doit être entendu que lorsque $R^2$ et éventuellement $R^1$ représente un composé coloré, il s'agit d'un résidu d'un composé coloré suite à la réaction d'un composé coloré avec la fonction XH. De préférence, les composés colorés de l'invention présentent une fonction permettant leur réaction avec la fonction XH menant ainsi à un résidu du composé coloré, de préférence cette fonction est la fonction COOH neutralisée ou non.

De préférence, pour les composés de formule (I) selon l'invention, $R^1$ représente un groupe méthyle, éthyle, propyle ou butyle, de préférence méthyle.

De préférence, pour les composés de formule (I) selon l'invention, A représente un groupe $-CH_2-CH_2-$.
De préférence, pour les composés de formule (I) X est O ou N, de préférence O.
De préférence, pour les composés de formule (I) n représente un entier compris entre 1 et 500.
De préférence, pour les composés de formule (I) selon l'invention :

$R^1$ représente un groupe méthyle, éthyle, propyle ou butyle ; et/ou
A représente un groupe $-CH_2-CH_2-$, $-CH(CH_3)-CH_2-$ ; et/ou
X est O ou N, de préférence O ; et/ou
n représente un entier compris entre 1 et 500, de préférence entre 4 et 250.

De préférence, pour les composés de formule (I) selon l'invention :

$R^1$ représente un groupe méthyle ;
A représente un groupe $-CH_2-CH_2-$ ;
X est O ; et
n représente un entier compris entre 4 et 250.

De préférence, dans les composés de formule (I), le composé coloré est choisi parmi les groupes suivants :

- Les dérivés d'azobenzène possédant une fonction COOH neutralisée ou non réagissant avec la fonction XH ;
- Les dérivés d'acridine possédant une fonction COOH neutralisée ou non réagissant avec la fonction XH ;
- Les dérivés d'anthraquinone possédant une fonction COOH neutralisée ou non réagissant avec la fonction XH ;
- Les dérivés de phtalocyanines possédant une fonction COOH neutralisée ou non réagissant avec la fonction XH ;
- Les dérivés de quinone possédant une fonction COOH neutralisée ou non réagissant avec la fonction XH ;
- Les dérives d'indophénol possédant une fonction COOH neutralisée ou non réagissant avec la fonction XH ;
- Les dérivés d'oxazone possédant une fonction COOH neutralisée ou non réagissant avec la fonction XH ;
- Les dérivés de thiazine possédant une fonction COOH neutralisée ou non réagissant avec la fonction XH ;
- Les dérivés de xanthène possédant une fonction COOH neutralisée ou non réagissant avec la fonction XH ;
- Les dérivés de fluorone possédant une fonction COOH neutralisée ou non réagissant avec la fonction XH.

Par dérivés on entend, dans le cadre de la présente invention, des composés comprenant les fonctions mentionnées ci-dessus.
De préférence, dans les composés de formule (I), le composé coloré est choisi parmi les groupes suivants :

- Les dérivés d'azobenzène possédant une fonction COOH neutralisée ou non réagissant avec la fonction XH ;
- Les dérivés de xanthène possédant une fonction COOH neutralisée ou non réagissant avec la fonction XH.

De préférence, le composé coloré est choisi parmi :

- Les dérivés d'azobenzène possédant une fonction COOH neutralisée ou non réagissant avec la fonction XH ;
- Les dérivés de rhodamine possédant une fonction COOH neutralisée ou non réagissant avec la fonction XH ;
- Les dérivés de fluorescéine possédant une fonction COOH neutralisée ou non réagissant avec la fonction XH.

De préférence, le composé coloré est choisi parmi :

- Les dérivés d'azobenzène possédant une fonction COOH neutralisée ou non réagissant avec la fonction XH ;
- Les dérivés de rhodamine possédant une fonction COOH neutralisée ou non réagissant avec la fonction XH.

De préférence, le composé coloré $R^2$ et éventuellement $R^1$ est choisi parmi les composés de formule suivante :

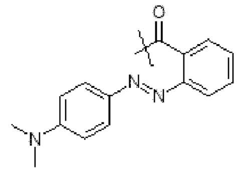

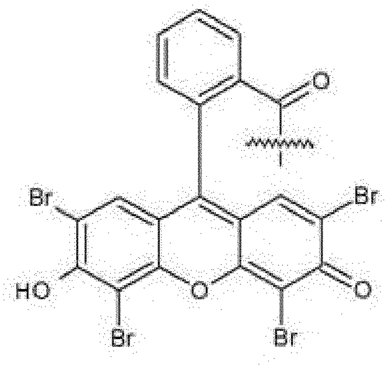

,

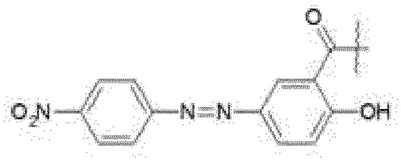

ou

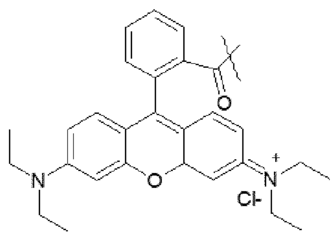

De préférence, le composé coloré $R^2$ et éventuellement $R^1$ est choisi parmi les composés de formule suivante :

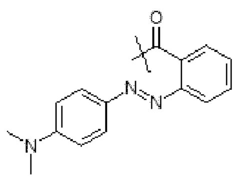

,

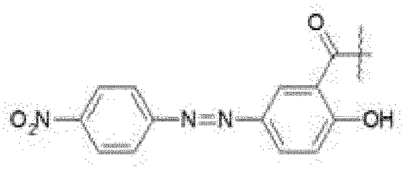

ou

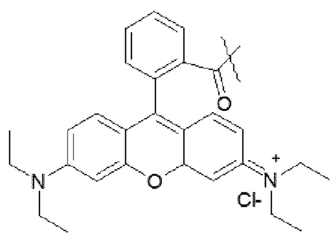

De façon particulièrement avantageuse, les inventeurs ont montré que les composés de formule (I) selon

l'invention s'intercalent dans les feuillets des argiles. Cette intercalation des composés de formule (I) se traduit par une diminution de l'intensité de la couleur du composé coloré. Cette diminution de l'intensité de la couleur du composé coloré permet de déterminer la quantité de composés de formule (I) intercalée ce qui permet de déterminer la quantité d'argile dans le sable.

De manière particulièrement avantageuse, les composés de formule (I) selon l'invention permettent spécifiquement de déterminer la quantité des argiles néfastes pour les superplastifiants, c'est-à-dire la quantité d'argiles intercalant les superplastifiants. De façon particulièrement avantageuse, les composés de formule (I) de l'invention ne vont pas s'intercaler dans les argiles n'intercalant pas les superplastifiants. Ainsi, les composés de formule (I) et les procédés mis en œuvre sont très spécifiques des argiles intercalant les superplastifiants.

Les composés de formule (I) peuvent être préparés par un procédé de préparation comprenant la réaction entre un composé de formule (II) et un composé coloré comprenant au moins une fonction réactive avec le groupe XH du composé de formule (II)

$$Y\text{-}(OA)_n\text{-}XH \qquad (II)$$

dans laquelle

Y représente H ou un groupe alkyle, linéaire ou ramifié, en C1 à C4;
A, X et n sont tels que définis pour les composés de formule (I).

Le procédé de la présente invention peut être réalisé à une température comprise entre 40°C et 200°C, de préférence entre 100°C et 185°C.

Les composés colorés sont choisis parmi les composés suivants :

- Les dérivés d'azobenzène possédant une fonction COOH neutralisée ou non réagissant avec la fonction XH ;
- Les dérivés d'acridine possédant une fonction COOH neutralisée ou non réagissant avec la fonction XH ;
- Les dérivés d'anthraquinone possédant une fonction COOH neutralisée ou non réagissant avec la fonction XH ;
- Les dérivés de phtalocyanines possédant une fonction COOH neutralisée ou non réagissant avec la fonction XH ;
- Les dérivés de quinone possédant une fonction COOH neutralisée ou non réagissant avec la fonction XH ;
- Les dérives d'indophénol possédant une fonction COOH neutralisée ou non réagissant avec la fonction XH ;
- Les dérivés d'oxazone possédant une fonction COOH neutralisée ou non réagissant avec la fonction XH ;
- Les dérivés de thiazine possédant une fonction COOH neutralisée ou non réagissant avec la fonction XH ;
- Les dérivés de xanthène possédant une fonction COOH neutralisée ou non réagissant avec la fonction XH ;
- Les dérivés de fluorone possédant une fonction COOH neutralisée ou non réagissant avec la fonction XH .

Par dérivés on entend, dans le cadre de la présente invention, des composés comprenant les fonctions mentionnées ci-dessus.

De préférence, dans les composés de formule (I), le composé coloré est choisi parmi les groupes suivants :

- Les dérivés d'azobenzène possédant une fonction COOH neutralisée ou non réagissant avec la fonction XH ;
- Les dérivés de xanthène possédant une fonction COOH neutralisée ou non réagissant avec la fonction XH.

De préférence, le composé coloré est choisi parmi :

- Les dérivés d'azobenzène possédant une fonction COOH neutralisée ou non réagissant avec la fonction XH ;
- Les dérivés de rhodamine possédant une fonction COOH neutralisée ou non réagissant avec la fonction XH.

De préférence, le composé coloré est choisi parmi :

- Les dérivés d'azobenzène possédant une fonction COOH neutralisée ou non réagissant avec la fonction XH ;
- Les dérivés de rhodamine possédant une fonction COOH neutralisée ou non réagissant avec la fonction XH ;
- Les dérivés de fluorescéine possédant une fonction COOH neutralisée ou non réagissant avec la fonction XH.

De préférence, le composé indicateur coloré est choisi parmi les composés suivant :

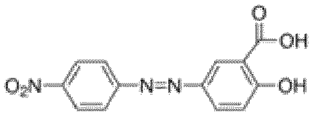 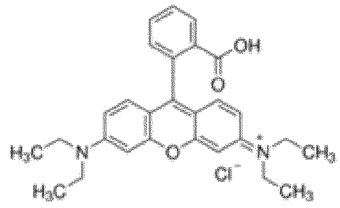 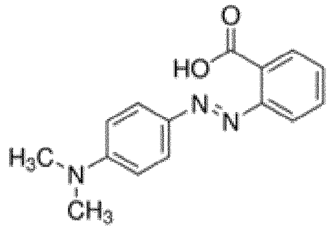

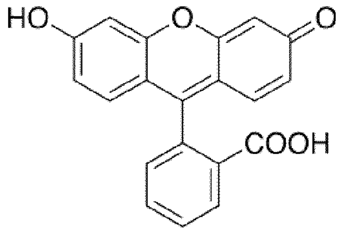

De préférence, le composé indicateur coloré est choisi parmi les composés suivant :

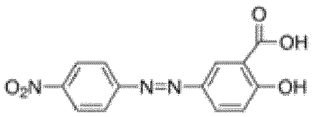 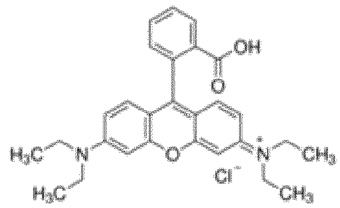 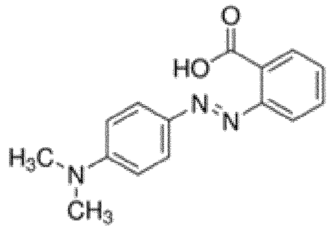

De préférence, les composés colorés sont les composés suivants :

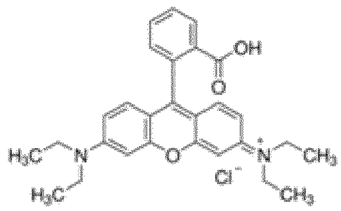 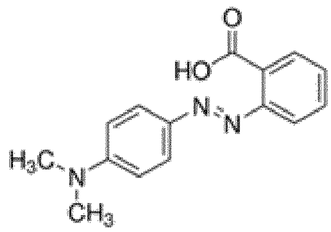

De préférence, dans le composé de formule (II) X est O.

De préférence, dans le composé de formule (II) Y est méthyle, éthyle, propyle ou butyle, de préférence méthyle.

De préférence, dans le composé de formule (II) A est $-CH_2-CH_2-$.

De préférence, dans le composé de formule (II) n représente un entier compris entre 1 et 500, de préférence entre 4 et 250.

De préférence, pour les composés de formule (II) selon l'invention :

$R^1$ représente un groupe méthyle, éthyle, propyle ou butyle, de préférence méthyle ; et/ou
A représente un groupe $-CH_2-CH_2-$ ; et/ou
X est O ; et/ou
n représente un entier compris entre 1 et 500, de préférence 4 et 250.

De préférence, pour les composés de formule (II) selon l'invention :

$R^1$ représente un groupe méthyle ;
A représente un groupe $-CH_2-CH_2-$ ;
X est O ;
n représente un entier compris entre 4 et 250.

De préférence, dans le procédé de l'invention, le ratio molaire composé de formule composé coloré/formule (II)

est compris entre 2 et 1, de préférence 1.

La présente invention concerne également l'utilisation d'un composé de formule (I) tel que décrit précédemment dans laquelle le composé de formule (I) est dans une composition (C) pour analyser et déterminer la quantité d'argile dans un sable, notamment pour déterminer par colorimétrie la quantité d'argile dans un sable, où la composition comprend également une solution tampon pH afin que la composition soit à un pH auquel le composé de formule (I) présente une adsorption des rayonnements dont les longueurs d'onde appartenant au domaine du visible. L'homme du métier est à même de déterminer le tampon pH à utiliser et le pH cible, l'objectif étant que la composition (C) soit à la couleur souhaitée. Par exemple, lorsque le composé coloré est le composé suivant :

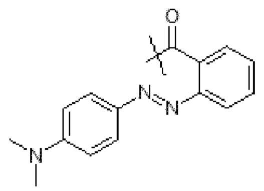

,

le pH de la composition (C) est compris entre 4 et 5,5, notamment par l'utilisation d'un tampon pH par exemple choisi dans le groupe constitué par de l'acide acétique/acétate de sodium/potassium ; dihydrogénophosphate/hydrogénophosphate de sodium/potassium, di-sodium/di-potassium ; les dihydrogénophosphate de sodium et/ou de potassium.

De préférence, la composition comprenant le composé de formule (I) ou la composition (C) est une composition aqueuse comprenant de 0,01 à 0,1% en poids de composé de formule (I).

Les inventeurs ont montré de façon avantageuse que les composés de l'invention pouvaient être utilisés pour déterminer la quantité d'argile dans un sable.

Ceci est particulièrement important pour estimer la consommation future de plastifiant ou superplastifiant, voire estimer le dosage de toute composition destinée à compenser l'effet négatif d'argiles dans une composition de béton. Comme indiqué précédemment, les composés de formule (I) selon l'invention s'intercalent dans les feuillets des argiles, à l'instar des molécules de superplastifiant ou de plastifiant. Cette intercalation des composés de formule (I) se traduit par une diminution de l'intensité de la couleur du composé coloré. Cette diminution de l'intensité de la couleur du composé coloré permet de déterminer la quantité de composés de formule (I) intercalés et donc consommés par le sable. Il est ensuite possible de déterminer la quantité d'argiles présente dans le sable. Cela permet de prévoir la quantité de superplastifiant ou plastifiant ou de tout composé AMAA à ajouter pour réduire voire supprimer les effets néfastes des argiles sur le maintien d'ouvrabilité.

De préférence, les AMAA sont décrits dans la demande de brevet EP1015398.

L'AMAA peut également par exemple être un composé de la gamme CHRYSO®Quad, préférentiellement CHRYSO®Quad 800.

La présente invention concerne également l'utilisation d'un composé de formule (I) selon l'invention pour déterminer la quantité de composé AMAA à ajouter à une composition de liant hydraulique, notamment pour réduire voire supprimer les effets néfastes des argiles présentent dans le sable sur le maintien d'ouvrabilité.

Ainsi, la présente invention concerne une méthode de détermination de la quantité d'argile dans un sable comprenant les étapes suivantes :

a) Fournir un composé de formule (I) selon l'invention ;
b) Prélever un échantillon du sable à analyser ;
c) Mélanger le composé de formule (I) avec l'échantillon de sable dans un récipient et agiter ;
d) Filtrer le mélange obtenu à l'étape c) ;
e) Déterminer en fonction de la couleur de la solution obtenue à l'étape d) la concentration en argiles dans le sable.

Il doit être entendu que la méthode selon l'invention permet de déterminer la quantité d'argile ayant un impact sur l'ouvrabilité des compositions de liant hydraulique, notamment les argiles impliquant une intercalation de superplastifiant dans les feuillets. Ainsi de façon préférée, la méthode selon l'invention permet la détermination de la quantité d'argile phyllosilicate dans un sable, de préférence les montmorillonites, encore plus préférentiellement les montmorillonites de sodium.

Dans un mode de réalisation, l'étape e) peut se faire par une détermination visuelle de la couleur et la corrélation de cette couleur à une plage de quantité d'argiles.

Dans un autre mode de réalisation, l'étape e) peut se faire par mesure photométrique de la couleur. Dans ce cas l'étape e) comprend une étape e1) de mesure photochimique de l'absorbance du filtrat obtenu à l'étape d) et une étape e2) de soustraction de la valeur obtenue à l'étape e) à la mesure photochimique de l'absorbance du composé de formule (I) et

de report de la valeur sur une courbe d'étalonnage pour déterminer le pourcentage en poids d'argile dans le sable. La méthode selon la présente invention peut donc également comprendre une étape préliminaire a0) de mesure photométrique de l'absorbance du composé de formule (I).

De préférence, l'agitation de l'étape c) est réalisée manuellement par retournement du récipient dans lequel se trouve le mélange, par exemple par retournement du récipient au moins 60 fois, par exemple 100 fois.

La mesure photométrique de l'absorbancedu composé de formule (I) et du filtrat obtenu à l'étape d) peut être réalisée par toute méthode connue de l'homme du métier et par tout matériel ayant une longueur d'onde compatible avec le composé indicateur coloré du composé de formule (I). Par exemple, cette mesure est effectuée avec un colorimètre, par exemple de longueur d'onde de laser comprise entre 400 et 700 nm, de préférence entre 475 et 600 nm.

Dans le cas où, le composé coloré est de formule :

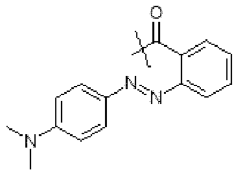

,

la mesure est de préférence réalisée avec un colorimètre présentant une longueur d'onde de laser comprise entre 400 et 700 nm, de préférence entre 475 et 600 nm.

Avant tout mesure photométrique de l'absorbance, la méthode selon l'invention peut comprendre avantageusement la mesure photométrique de l'absorbance d'un blanc. Le blanc peut être de l'eau ou, lorsque le composé de formule (I) est dans la composition (C) comprenant une solution tampon le blanc peut être réalisé avec cette solution tampon. La réalisation d'un blanc permet de s'affranchir de toute variation de mesure liée à tout élément extérieur au mélange à analyser, par exemple récipient comprenant le mélange à analyser.

L'étape d) de filtration du mélange obtenu à l'étape c) peut être réalisée de toute manière connue de l'homme du métier. De préférence, le mélange, à la fin de l'étape c) est laissé à décanter, de préférence entre 1 et 60 minutes, de préférence entre 1 et 30 minutes. Cette étape de décantation permet avantageusement aux particules les plus fines de retomber dans le fond du récipient et éviter de boucher le filtre. Le surnageant est ensuite prélevé avec tout matériel adéquat, par exemple avec une seringue, puis filtré, de préférence sur un filtre de porosité comprise entre 0,25 et 5 $\mu$m, de préférence entre 0,25 et 2 $\mu$m. De préférence la quantité de filtrat à prélever pour effectuer la mesure photométrique dépend de l'appareil utilisé et pourra être déterminée par l'homme du métier, par exemple le volume est d'au moins 10 ml.

De préférence, l'échantillon de sable prélevé correspond à une masse comprise entre 10 et 100 g. De préférence, la quantité de composition comprenant le composé de formule (I) ou de composition (C) ajoutée à l'échantillon de sable est d'au moins 25 ml, de préférence comprise entre 25 et 100 ml.

La méthode de l'invention peut également comprendre une étape d'ajustement du pH avant l'étape e) afin d'être dans la gamme de valeur de pH permettant de voir la couleur du composé coloré.

Afin de déterminer la quantité en argile contenue dans l'échantillon de sable, il convient de réaliser une courbe d'étalonnage. Cette courbe d'étalonnage peut être réalisée par toute méthode connue de l'homme du métier.

Notamment, la courbe d'étalonnage peut être obtenue par exemple :

- par mesure photométrique ou détection visuelle du changement de couleur d'un sable exempt d'argiles (par exemple sable AFNOR pur) et d'un sable exempt d'argiles additionné de différentes quantités connues d'argiles;
- par mesure du carbone organique totale (COT) d'un sable exempt d'argiles (par exemple sable AFNOR pur) et d'un sable exempt d'argiles additionné de différentes quantités connues d'argiles;
- par mesure du carbone organique total (COT) d'un sable exempt d'argiles (par exemple sable AFNOR pur) et de sables comprenant des argiles.

Sans vouloir être lié par une quelconque théorie, la consommation de poly(alkylène) glycol par les argiles, notamment Montmorillonite est déterminée par différence entre la quantité de poly(alkylène) glycol en solution avant introduction du sable dans la solution de poly(alkylène) glycol et après 5 min de contact entre le sable et cette solution. La teneur en COT dans le filtrat d'une suspension de sable sans poly(alkylène) glycol est également mesurée et sert de blanc aux mesures de COT.

La mesure de COT est effectuée sur les solutions initiales et les filtrats avec l'analyseur SHIMADZU TOC-VCPN. Le COT est calculé par différence entre la quantité en carbone total (obtenue par carbonisation de la solution et mesure de la quantité de $CO_2$ dégagée à l'infra-rouge) et la quantité de carbone inorganique (obtenue par acidification de la solution à pH < 1 et dégagement du $CO_2$ dissout par bullage à l'air synthétique). La quantité de poly(alkylène) glycol consommée est

calculée par différence entre ce qui a été introduit dans la solution initiale et ce qui est mesuré dans les filtrats. Dans le cadre de la présente invention les mesures COT sont réalisées après filtration du surnageant résultat de la mise en contact du sable avec la solution de la composition comprenant le composé de formule (I) ou de la composition (C).

La courbe d'étalonnage permettant de réaliser l'étape e) selon l'invention peut être obtenue de la manière suivante :

- on ajoute une quantité connue de composition comprenant le composé de formule (I) ou de composition (C) à une quantité connue de sable exempt d'argiles (par exemple sable AFNOR pur), on mélange, on filtre (le mélange et la filtration peuvent être de préférence identique aux étapes c) et d) mentionnées ci-dessus) et on regarde la couleur du filtrat obtenu ;
- on ajoute une quantité connue de composition comprenant le composé de formule (I) ou de composition (C) à différents mélanges comprenant une quantité connue de sable exempt d'argiles (par exemple sable AFNOR pur) et une quantité connue d'argiles, on mélange, on filtre (le mélange et la filtration peuvent être de préférence identique aux étapes c) et d) mentionnées ci-dessus) et on regarde les différentes couleurs des filtrats obtenus ;
- on détermine un gradient de couleur correspondant à différentes plages de concentration en argiles dans le sable.

La courbe d'étalonnage permettant de réaliser l'étape e) selon l'invention peut également être obtenue de la manière suivante :

- on réalise une mesure photométrique de la composition comprenant le composé de formule (I) ou de composition (C);
- on ajoute une quantité connue de composition comprenant le composé de formule (I) ou de composition (C) à une quantité connue de sable exempt d'argiles (par exemple sable AFNOR pur), on mélange, on filtre (le mélange et la filtration peuvent être de préférence identique aux étapes c) et d) mentionnées ci-dessus) et on réalise une mesure photométrique du filtrat obtenu à laquelle on déduit la valeur de la mesure photométrique de la composition comprenant le composé de formule (I) ou de composition (C);
- on ajoute une quantité connue de composition comprenant le composé de formule (I) ou de composition (C) à différents mélanges comprenant une quantité connue de sable exempt d'argiles (par exemple sable AFNOR pur) et une quantité connue d'argiles, on mélange, on filtre (le mélange et la filtration peuvent être de préférence identique aux étapes c) et d) mentionnées ci-dessus) et on réalise une mesure photométrique des filtrats obtenus auxquelles on déduit la valeur de la mesure photométrique de la composition comprenant le composé de formule (I) ou de composition (C);
- on détermine la courbe de l'absorbance en fonction de la concentration en argiles dans le sable.

La courbe d'étalonnage permettant de réaliser l'étape e) selon l'invention peut également être obtenue de la manière suivante :

- on réalise une mesure photométrique de la composition comprenant le composé de formule (I) ou de composition (C);
- on mesure le COT d'une quantité connue de sable exempt d'argiles (par exemple sable AFNOR pur) ;
- on ajoute une quantité connue de composition comprenant le composé de formule (I) ou de composition (C) selon l'invention à une quantité connue de sable exempt d'argiles (par exemple sable AFNOR pur), on mélange, on filtre (le mélange et la filtration peuvent être de préférence identique aux étapes c) et d) mentionnées ci-dessus) et on réalise une mesure photométrique du filtrat obtenu à laquelle on déduit la valeur de la mesure photométrique de la composition comprenant le composé de formule (I) ou de composition (C), on relie la valeur COT avec la valeur de photométrie ce qui permet d'avoir une valeur d'absorbance pour un sable exempt d'argiles ;
- on mesure le COT d'une quantité connue d'au moins deux sables commerciaux (par exemple sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan);
- on ajoute une quantité connue de composition comprenant le composé de formule (I) ou de composition (C) à au moins deux sables commerciaux (par exemple sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan) on mélange, on filtre (le mélange et la filtration peuvent être de préférence identique aux étapes c) et d) mentionnées ci-dessus) et on réalise une mesure photométrique des différents filtrats obtenus auxquelles on déduit la valeur de la mesure photométrique de la composition comprenant le composé de formule (I) ou de composition (C), on relie la valeur COT avec la valeur de photométrie obtenue ce qui permet d'avoir une valeur d'absorbance pour une concentration en argile connue ;
- on détermine la courbe l'absorbance en fonction de la concentration en argiles dans le sable.

La relation entre la valeur COT (MPEG consommé) et la concentration en argile (Eq Mnt) est la suivante :

$$EqMnt = \frac{m_{MPEG\ consommé} + 0{,}3648}{1{,}1943} \quad (1)$$

La courbe d'étalonnage permettant de réaliser l'étape e) selon l'invention peut également être obtenue de la manière suivante :

- on mesure le COT d'une quantité connue de sable exempt d'argiles (par exemple sable AFNOR pur) ;
- on ajoute une quantité connue de composition comprenant le composé de formule (I) ou de composition (C) à une quantité connue de sable exempt d'argiles (par exemple sable AFNOR pur), on mélange, on filtre (le mélange et la filtration peuvent être de préférence identique aux étapes c) et d) mentionnées ci-dessus) et on regarde la couleur du filtrat obtenu ce qui permet d'avoir la couleur d'un sable exempt d'argiles ;
- on mesure le COT d'une quantité connue d'au moins deux sables commerciaux (par exemple sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan);
- on ajoute une quantité connue de composition comprenant le composé de formule (I) ou de composition (C) à au moins deux sables commerciaux (par exemple sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan) on mélange, on filtre (le mélange et la filtration peuvent être de préférence identique aux étapes c) et d) mentionnées ci-dessus) et on regarde la couleur des filtrats obtenus, on relie la valeur COT avec la gamme de couleur obtenu ce qui permet d'avoir un gradient de couleur en fonction de la plage de concentration en argile.

La présente invention permet avantageusement de prévoir le dosage en composé AMAA à utiliser pour limiter voire supprimer l'effet néfaste des argiles notamment sur la réduction d'eau et le maintien d'ouvrabilité. Pour cela, il convient de construire une courbe de corrélation permettant de relier le dosage nécessaire en AMAA en fonction de la couleur ou de l'absorbance (mesure photométrique) obtenu à l'étape e).

Cette courbe de corrélation peut être obtenue en déterminant la quantité d'AMAA à utiliser en fonction de la concentration en argile dans le sable, la relation entre la concentration en argile dans le sable et la couleur ou l'absorbance (mesure photométrique) obtenu à l'étape e) étant détaillée ci-dessus.

La courbe de corrélation entre la quantité d'AMAA à utiliser en fonction de la concentration en argile dans le sable peut être réalisée de la manière suivante :

- on mesure l'étalement à 5 minutes (T5) et le maintien d'un mortier de référence obtenu avec du sable AFNOR ;
- on mesure l'étalement à 5 minutes (T5) et le maintien d'au moins deux mortiers obtenus avec des sables commerciaux différents (par exemple sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan);
- on ajoute l'AMAA aux mortiers obtenus avec des sables commerciaux différents de façon à obtenir un étalement à 5 minutes (T5) et un maintien similaire à celui obtenu pour le mortier de référence ;
- on en déduit la courbe de la quantité d'AMAA à ajouter en fonction de la concentration en argiles dans le sable.

Il est ensuite possible avec les courbes d'étalonnage décrites ci-dessus de déterminer la courbe de la quantité d'AMAA en fonction de la couleur ou valeur d'absorbance (mesure photométrique) obtenu à l'étape e). Cette courbe est de préférence construite en fonction du liant hydraulique mis en œuvre dans le mortier.

L'étalement peut notamment être évalué comme suit :
Un moule sans fond de forme tronconique, de reproduction à l'échelle 0,5 du cône d'Abrams (voir norme NF 18-451, 1981) de dimensions suivantes diamètre du cercle supérieur = 5cm, diamètre du cercle de la base inférieure = 10 cm, hauteur 15 cm. Après malaxage du mortier renfermant le polymère, le moule est rempli et la surface supérieure du cône est arasée. Le cône est soulevé verticalement et l'étalement est mesuré à 90° avec un mètre à ruban.

Il est possible d'ajuster le pH des filtrats avant lecture de la couleur (à l'œil ou par spectrométrie) afin d'être dans la gamme de valeur de pH permettant de voir la couleur du composé coloré.

Avant tout mesure photométrique de l'absorbance, il est conseillé de réaliser la mesure photométrique de l'absorbance d'un blanc. Le blanc peut être de l'eau ou la solution tampon utilisée dans la composition (C). La réalisation d'un blanc permet de s'affranchir de toute variation de mesure liée à tout élément extérieur au mélange à analyser, par exemple récipient comprenant le mélange à analyser.

La présente invention concerne également un kit pour la mise en œuvre de la méthode selon l'invention, comprenant :

- Un récipient muni d'un bouchon comprenant le composé de formule (I) selon l'invention ;
- Un récipient pour le dosage de l'échantillon de sable ;
- Un moyen de prélèvement de liquide ;
- Un moyen de filtration ;
- Un récipient pour la récupération du filtrat ;
- Une courbe d'étalonnage permettant de relier la couleur à la concentration en argile du sable et/ou une courbe de corrélation permettant de relier la couleur à la quantité d'AMAA à utiliser.

De préférence, le récipient pour la récupération du filtrat est un récipient transparent permettant de voir la couleur du filtrat. De préférence, le récipient pour la récupération du filtrat est adapté à l'analyse photométrique de l'absorbance.
De préférence l'échantillon de sable est versé dans le récipient muni d'un bouchon comprenant le composé de formule (I) selon l'invention.
De préférence, le moyen de prélèvement de liquide est une seringue.
De préférence, le moyen de filtration est un filtre seringue présentant une taille de porosité préférence comprise entre 0,25 et 5 $\mu$m, de préférence entre 0,25 et 2 $\mu$m.
De préférence, le kit selon l'invention comprend un appareil de mesure photométrique de l'absorbance. De préférence, l'appareil permettant la mesure photométrique de l'absorbance est un appareil de colorimétrie présentant une longueur d'onde de laser comprise entre 400 et 700 nm, de préférence entre 475 et 600 nm.
Le kit selon l'invention peut également comprendre une solution d'acide ou de base qui permet avantageusement d'ajuster le pH pour être dans la gamme de valeur de pH permettant de voir la couleur du composé coloré.
La présente invention concerne également une méthode de détermination de la quantité d'AMAA à ajouter dans un sable pour son utilisation dans une composition de liant hydraulique comprenant les étapes suivantes :

i) Fournir un composé de formule (I) selon l'invention ;
ii) Prélever un échantillon du sable à analyser ;
iii) Mélanger le composé de formule (I) avec l'échantillon de sable dans un récipient et agiter ;
iv) Filtrer le mélange obtenu à l'étape iii) ;
v) Déterminer en fonction de la couleur de la solution obtenue à l'étape iv) la quantité d'AMAA à ajouter.

L'étape ii), iii) et iv) pouvant être réalisée de manière identique aux étapes b), c) et d) décrites ci-dessus.
L'étape v) pouvant être réalisée de manière identique à l'étape e) décrite ci-dessus, les courbes de corrélation entre la valeur d'absorbance et la quantité d'AMAA à ajouter pouvant être réalisée comme décrit précédemment.
De préférence, la présente invention concerne un composé de formule (I)

$$R^1\text{-}(OA)_n\text{-}XR^2 \qquad \text{(I)}$$

dans laquelle

$R^1$ représente un groupe alkyle en C1 à C4, linéaire ou ramifié, ou un dérivé d'azobenzène possédant une fonction COOH neutralisée ou non réagissant avec la fonction XH ;
$R^2$ représente un dérivé d'azobenzène possédant une fonction COOH neutralisée ou non réagissant avec la fonction XH;
A, chacun identique ou différent, représente indépendamment un groupe $-CH_2-CH_2-$ ou un groupe $-CH(CH_3)-CH_2-$ ;
n représente un entier compris entre 1 et 500, de préférence compris entre 4 et 250
X est O ou NH.

De préférence, $R^1$ représente un groupe alkyle en C1 à C4, linéaire ou ramifié, et $R^2$ représente :

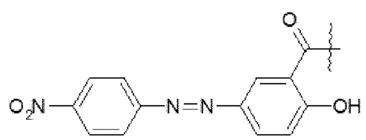

ou

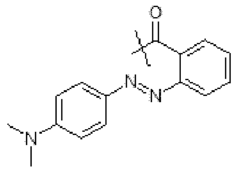

La présente invention va maintenant être décrite à l'aide d'exemples non limitatifs.

[Fig 1] La figure 1 représente la courbe de corrélation entre la concentration en AMAA à utiliser et la valeur d'absorbance mesurée pour la composition (C1) de l'exemple 1.
[Fig 2] La figure 2 représente la courbe de corrélation entre la concentration en AMAA à utiliser et la valeur d'absorbance mesurée pour la composition (C2) de l'exemple 2.

**Exemple 1** : **Synthèse d'une composition (C1)**

Dans un ballon bicol munis d'un Dean Stark, 8,08g de composé A, 140,80g de MPEG 5000 et 1,13g de lessive de soude (extrait sec de 50%) sont ajoutés puis mis sous agitation. Le milieu réactionnel est porté à 165°C sous un vide de 20 mbars. Dans ces conditions, le milieu réactionnel est conservé pendant 6h. La température est abaissée à température ambiante puis le milieu réactionnel est dilué avec un tampon (acide acétique/acétate de sodium 0,1M) pour obtenir l'extrait sec désiré (0,04% massique).
Composé A :

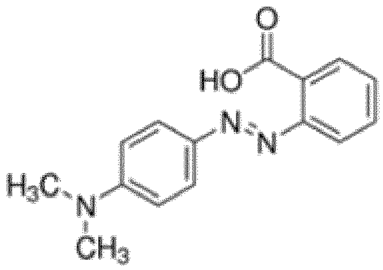

**Exemple 2** : **Synthèse d'une composition (C2)**

Dans un ballon bicol munis d'un Dean Stark, 3,04g de composé B, 31,71g de MPEG 5000 et 0,25g de lessive de soude (extrait sec de 50%) sont ajoutés puis mis sous agitation. Le milieu réactionnel est porté à 165°C sous un vide de 20 mbars. Dans ces conditions, le milieu réactionnel est conservé pendant 6h. La température est abaissée à température ambiante puis le milieu réactionnel est dilué pour obtenir l'extrait sec désiré (0,1% massique).
Composé B :

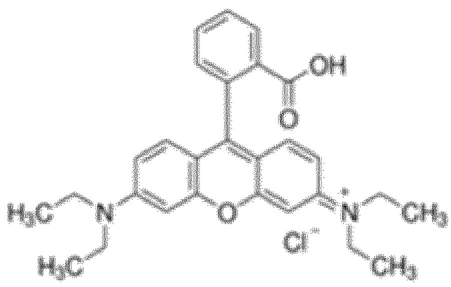

**Exemple 3** : **Synthèse d'une composition (C3)**

Dans un ballon bicol munis d'un Dean Stark, 7,60g de composé C, 141,27g de MPEG 5000 et 1,13g de lessive de soude (extrait sec de 50%) sont ajoutés puis mis sous agitation. Le milieu réactionnel est porté à 165°C sous un vide de 20 mbars. Dans ces conditions, le milieu réactionnel est conservé pendant 6h. La température est abaissée à température ambiante puis le milieu réactionnel est dilué pour obtenir l'extrait sec désiré (0,04% massique).
Composé C :

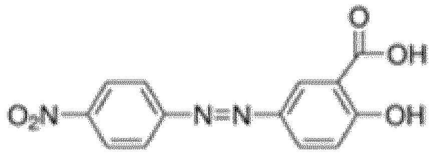

**Exemple 4** : **Réalisation d'une droite reliant la concentration en argile à la valeur COT**

La consommation de MPEG5000 par les argiles, notamment Montmorillonite est déterminée par différence entre la quantité de MPEG 5000 en solution avant introduction du sable dans la solution de polymère et après 5 min de contact entre le sable et cette solution. La teneur en COT dans le filtrat d'une suspension de sable sans polymères est également mesurée et sert de blanc aux mesures de COT.

La mesure de COT est effectuée sur les solutions initiales et les filtrats avec l'analyseur SHIMADZU TOC-VCPN. Le COT est calculé par différence entre la quantité en carbone total (obtenue par carbonisation de la solution et mesure de la quantité de $CO_2$ dégagée à l'infra-rouge) et la quantité de carbone inorganique (obtenue par acidification de la solution à pH < 1 et dégagement du $CO_2$ dissout par bullage à l'air synthétique). La quantité de MPEG5000 consommée est calculée par différence entre ce qui a été introduit dans la solution initiale et ce qui est mesuré dans les filtrats.

La consommation de MPEG 5000 a été mesurée sur différents sables prélevés sur le terrain pour des dosages initiaux 0,4. L'équivalent en montmorillonite (EqMnt) est calculé à partir de l'équation suivante :

$$EqMnt = \frac{m_{MPEG\ consommé} + 0{,}3648}{1{,}1943} \quad (1)$$

Les sables prélevés sur le terrain sont les suivants : sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan Cela permet d'obtenir une droite reliant la concentration en argile à la valeur COT.

**Exemple 5** : **Réalisation d'une courbe d'étalonnage de la valeur d'absorbance en fonction de la concentration en argile**

50 g de sable AFNOR (exempt d'argiles) sont prélevés et introduits dans un récipient comprenant 50 g d'une composition (C1) de l'exemple 1. Le mélange est agité 30 secondes puis décanté environ 2 minutes pour que les particules les plus fines retombent afin d'éviter le bouchage du filtre. 10 ml de surnageant sont prélevés puis filtré à l'aide d'un filtre seringue en fibre de verre de 1 $\mu$m. Le filtrat obtenu est récupéré dans un tube en verre pour mesure photométrique de l'absorbance (A1). Avant la mesure de l'absorbance 2 gouttes d'HCl sont ajoutées au filtrat. Avant la mesure de l'absorbance du filtrat un blanc est réalisé avec une solution tampon (acide acétique/acétate de sodium 0,1M). La mesure de l'absorbance de la composition (C1) est effectuée et cette valeur est déduite la mesure de l'absorbance (A1).

Le même protocole est mis en œuvre avec les sables suivants sable Osman, sable Signes, sable St Marthe, sable Lecieux, sable Fulchiron, sable Vernou, sable Vesseny, sable Goutrens, sable Bernières, sable Inerti Salinello, sable Siegwart, sable TRK, sable Sail s/s Couzan. Les mesures d'absorbances (An) sont réalisées. Avant la mesure de l'absorbance 2 gouttes d'HCl sont ajoutées aux filtrats. Avant la mesure de l'absorbance du filtrat un blanc est réalisé avec une solution tampon (acide acétique/acétate de sodium 0,1M). La mesure de l'absorbance de la composition (C1) est effectuée et cette valeur est déduite des mesures d'absorbance (An).

Une corrélation avec la droite obtenue à l'exemple 4 permet d'obtenir une courbe de la valeur d'absorbance en fonction de la concentration en argile.

Un protocole similaire est mis en œuvre avec la composition (C2) de l'exemple 2.

**Exemple 6** : **Réalisation d'une droite de corrélation entre la quantité d'AMAA à ajouter à la valeur d'absorbance**

4 mortiers sont réalisés selon la composition suivante :

- 624,9 g de ciment CEM I 52,5N CE CP2 NF SPLC
- E/C = 0,6
- 734,98 $cm^3$ de sable

Les courbes granulaires des mortiers sont homogénéisées en associant aux sables d'intérêt du sable Fulchiron

qui ne contient pas d'argile (cela permet de ne pas subir l'influence de la courbe granulaire et par conséquent de n'étudier que l'effet des argiles), dans les proportions volumiques suivantes :

[Tableau 1]

| | Sable d'intéret | Fraction volumique du sable d'intérêt (% poids) | Fraction volumique du sable Fulchiron (% poids) |
|---|---|---|---|
| Mortier 1 - ref | AFNOR | 69,5 | 30,5 |
| Mortier 2 | Sail sous Couzan | 49 | 51 |
| Mortier 3 | Siegwart | 78,6 | 21,4 |
| Mortier 4 | Vernou | 59,3 | 40,7 |

La mesure d'étalement à T5 et de maintien a été réalisée pour le mortier 1 de référence ne présentant pas d'argile.

L'étalement est évalué comme suit :

Un moule sans fond de forme tronconique, de reproduction à l'échelle 0,5 du cône d'Abrams (voir norme NF 18-451, 1981) de dimensions suivantes diamètre du cercle supérieur = 5cm, diamètres du cercle de la base inférieure = 10 cm, hauteur 15 cm. Après malaxage du mortier renfermant le polymère, le moule est rempli et la surface supérieure du cône est arasée. Le cône est soulevé verticalement et l'étalement est mesuré à 90° avec un mètre à ruban.

Les mesures d'étalement à T5 et de maintien ont été réalisées pour les mortiers 2 à 4.

L'AMAA (CHRYSO®Quad 800) a été ajouté aux mortiers 2 à 4 jusqu'à obtenir des mesures d'étalement à T5 et de maintien similaire à celle du mortier 1 de référence.

Les résultats sont les suivants :

[Tableau 2]

| | Dosage préconisé en AMAA (% PdS d'intérêt) |
|---|---|
| Mortier 1 - ref | - |
| Mortier 2 | 0,3 |
| Mortier 3 | 0,25 |
| Mortier 4 | 0,15 |

On en déduit ainsi, une droite de la quantité en AMAA à utiliser en fonction du sable et donc de la quantité en argile (la quantité en argile de chacun des sables ayant été obtenue à l'exemple 4) et par corrélation avec les courbes des exemples 4 et 5 on en déduit la courbe de corrélation de la concentration en AMAA à utiliser en fonction de la valeur en absorbance.

Les deux courbes obtenues pour les compositions (C1) et (C2) des exemples 1 et 2 sont données respectivement aux figures 1 et 2.

Les courbes d'étalonnages dépendent des ciments mis en œuvre.

**Exemple 7 : Mode opératoire du test colorimétrique** :

- Fournir un récipient (bouteille de 100mL) muni d'un bouchon comprenant la 50 ml de la composition (C) de l'invention;
- Introduction du sable prélevé dans un récipient de 35mL (soit environ 10g de sable);
- Agitation durant environ 30 secondes puis décantation ;
- Prélèvement du liquide surnageant (seringue de 10mL) ;
- Filtration (filtre seringue en fibre de verre 1μm) dans un récipient adapté à l'analyse photométrique de l'absorbance ;
- Mesure photométrique de l'absorbance via un appareil permettant des mesures à une longueur d'onde de 525nm;
- Report de la valeur lue sur l'appareil dans une courbe d'équivalence permettant de relier la valeur de l'absorbance au dosage requis en AMAA.

## Revendications

1. Utilisation de composé de formule (I) pour déterminer la quantité d'argile intercallant les superplastifiants dans un sable et/ou pour déterminer la quantité de composé Agent Modificateur de l'Activité de l'Argile AMAA à ajouter à une composition de liant hydraulique utilisant un sable

$$R^1\text{-}(OA)_n\text{-}XR^2 \qquad (I)$$

dans laquelle

$R^1$ représente un groupe alkyle en C1 à C4, linéaire ou ramifié, ou un composé coloré ;
$R^2$ représente un composé coloré;
A, chacun identique ou différent, représente indépendamment un groupe $-CH_2-CH_2-$ ou un groupe $-CH(CH_3)-CH_2-$ ;
n représente un entier compris entre 1 et 500, de préférence compris entre 4 et 250 X est O ou NH.

2. Utilisation selon la revendication 1, dans lequel

$R^1$ représente un groupe méthyle, propyle, éthyle ou butyle ; et/ou
A représente un groupe $-CH_2-CH_2-$, $-CH(CH_3)-CH_2-$ ; et/ou
X est O ou NH, de préférence O ; et/ou
n représente un entier compris entre 1 et 500, de préférence entre 4 et 250.

3. Utilisation selon la revendication 1 ou 2, dans lequel le composé coloré est choisi parmi les groupes suivants :

- Les dérivés d'azobenzène possédant une fonction COOH neutralisée ou non réagissant avec la fonction XH ;
- Les dérivés d'acridine possédant une fonction COOH neutralisée ou non réagissant avec la fonction XH ;
- Les dérivés d'anthraquinone possédant une fonction COOH neutralisée ou non réagissant avec la fonction XH ;
- Les dérivés de phtalocyanines possédant une fonction COOH neutralisée ou non réagissant avec la fonction XH ;
- Les dérivés de quinone possédant une fonction COOH neutralisée ou non réagissant avec la fonction XH ;
- Les dérives d'indophénol possédant une fonction COOH neutralisée ou non réagissant avec la fonction XH ;
- Les dérivés d'oxazone possédant une fonction COOH neutralisée ou non réagissant avec la fonction XH ;
- Les dérivés de thiazine possédant une fonction COOH neutralisée ou non réagissant avec la fonction XH ;
- Les dérivés de xanthène possédant une fonction COOH neutralisée ou non réagissant avec la fonction XH ;
- Les dérivés de fluorone possédant une fonction COOH neutralisée ou non réagissant avec la fonction XH.

4. Utilisation selon la revendication 1 ou 2, dans lequel le composé coloré est choisi parmi les groupes suivants :

- Les dérivés d'azobenzène possédant une fonction COOH neutralisée ou non réagissant avec la fonction XH ;
- Les dérivés de xanthène possédant une fonction COOH neutralisée ou non réagissant avec la fonction XH.

5. Utilisation selon la revendication 1 ou 2, dans lequel le composé coloré est choisi parmi les groupes suivants :

- Les dérivés d'azobenzène possédant une fonction COOH neutralisée ou non réagissant avec la fonction XH ;
- Les dérivés de rhodamine possédant une fonction COOH neutralisée ou non réagissant avec la fonction XH.

6. Utilisation selon la revendication 1 ou 2, dans lequel le composé coloré est choisi parmi les groupes suivants :

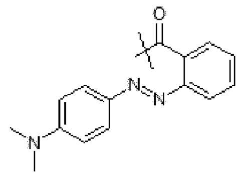

,

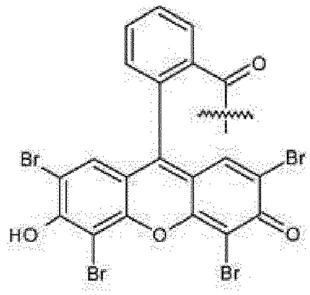

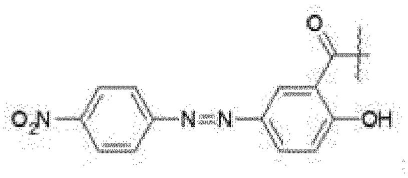

ou

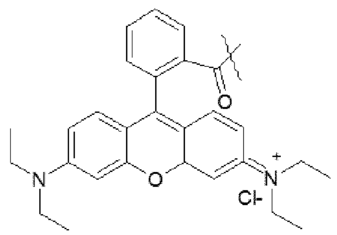

7. Utilisation selon la revendication 1 ou 2, dans lequel le composé coloré est choisi parmi les groupes suivants :

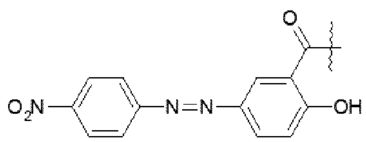

;

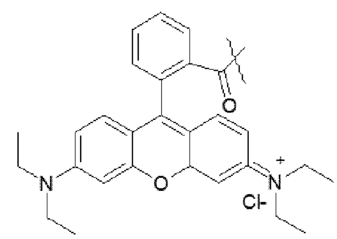

;

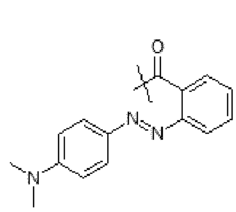

8. Utilisation selon l'une quelconque des revendications 1 à 7 dans laquelle le composé de formule (I) est dans une composition (C) qui comprend en outre un tampon pH.

9. Méthode de détermination de la quantité d'argile intercallant les superplastifiants dans un sable comprenant les étapes suivantes :

a) Fournir un composé de formule (I) tel que défini selon l'une quelconque des revendications 1 à 7 ;
b) Prélever un échantillon du sable à analyser ;
c) Mélanger le composé de formule (I) avec l'échantillon de sable dans un récipient et agiter ;
d) Filtrer le mélange obtenu à l'étape c) ;
e) Déterminer en fonction de la couleur de la solution obtenue à l'étape d) la concentration en argiles intercallant les superplastifiants dans le sable.

10. Méthode selon la revendication 9, dans laquelle l'étape e) se fait par une détermination visuelle de la couleur et la corrélation de cette couleur à une plage de quantité d'argiles intercallant les superplastifiants ou par mesure photométrique comportant alors les étapes e1) de mesure photochimique de l'absorbance du filtrat obtenu à l'étape d) et une étape e2) de soustraction de la valeur obtenue à l'étape e) à la mesure photochimique de l'absorbance du composé de formule (I) et de report de la valeur sur une courbe d'étalonnage pour déterminer le pourcentage en poids d'argile intercallant les superplastifiants dans le sable.

11. Méthode de détermination de la quantité d' Agent Modificateur de l'Activité de l'Argile AMAA à ajouter dans un sable pour son utilisation dans une composition de liant hydraulique comprenant les étapes suivantes :

i) Fournir un composé de formule (I) tel que défini selon l'une quelconque des revendications 1 à 7 ;
ii) Prélever un échantillon du sable à analyser ;
iii) Mélanger le composé de formule (I) avec l'échantillon de sable dans un récipient et agiter ;
iv) Filtrer le mélange obtenu à l'étape iii) ;
v) Déterminer en fonction de la couleur de la solution obtenue à l'étape iv) la quantité d'AMAA à ajouter.

12. Kit pour la mise en œuvre de la méthode selon les revendications 9 à 11, comprenant :

- Un récipient muni d'un bouchon comprenant un composé de formule (I) tel que défini selon l'une quelconque des revendications 1 à 7 ;
- Un récipient pour le dosage de l'échantillon de sable ;
- Un moyen de prélèvement de liquide ;
- Un moyen de filtration ;
- Un récipient pour la récupération du filtrat ;
- Une courbe d'étalonnage permettant de relier la couleur à la concentration en argile intercallant les superplastifiants du sable et/ou une courbe de corrélation permettant de relier la couleur à la quantité d'Agent Modificateur de l'Activité de l'Argile AMAA à utiliser.

13. Kit selon la revendication 12 comprenant en outre un appareil permettant la mesure photométrique de l'absorbance.

**Patentansprüche**

1. Verwendung einer Verbindung der Formel (I) zur Bestimmung der Tonmenge, die das Fließmittel in einem Sand interkaliert, und/oder zur Bestimmung der Menge einer Verbindung an Ton-Aktivitätsmodifikator-Mittel AMAA, die einer hydraulischen Bindemittelzusammensetzung mit Sand zuzusetzen ist

$$R^1\text{-}(OA)_n\text{-}XR^2 \qquad (I)$$

wobei

$R^1$ eine lineare oder verzweigte C1- bis C4-Alkylgruppe oder eine farbige Verbindung darstellt;
$R^2$ eine farbige Verbindung darstellt;
A, jeweils gleich oder verschieden, unabhängig voneinander eine Gruppe $-CH_2-CH_2-$ oder eine Gruppe $-CH(CH_3)-CH_2-$ darstellt;
n eine Ganzzahl zwischen 1 und 500, vorzugsweise zwischen 4 und 250, darstellt;
X O oder NH ist.

**2.** Verwendung nach Anspruch 1, wobei

$R^1$ eine Methyl-, Propyl-, Ethyl- oder Butylgruppe darstellt; und/oder
A eine Gruppe $-CH_2-CH_2-$, $-CH(CH_3)-CH_2-$ darstellt; und/oder
X O oder NH, vorzugsweise O, ist; und/oder
n eine Ganzzahl zwischen 1 und 500, vorzugsweise zwischen 4 und 250, darstellt.

**3.** Verwendung nach Anspruch 1 oder 2, wobei die farbige Verbindung aus den folgenden Gruppen ausgewählt ist:

- den Azobenzolderivaten, die eine neutralisierte COOH-Funktion besitzen oder nicht mit der XH-Funktion reagieren;
- den Acridinderivaten, die eine neutralisierte COOH-Funktion besitzen oder nicht mit der XH-Funktion reagieren;
- den Anthrachinonderivaten, die eine neutralisierte COOH-Funktion besitzen oder nicht mit der XH-Funktion reagieren;
- den Phthalocyaninderivaten, die eine neutralisierte COOH-Funktion besitzen oder nicht mit der XH-Funktion reagieren;
- den Chinonderivaten, die eine neutralisierte COOH-Funktion besitzen oder nicht mit der XH-Funktion reagieren;
- den Indophenolderivaten, die eine neutralisierte COOH-Funktion besitzen oder nicht mit der XH-Funktion reagieren;
- den Oxazonderivaten, die eine neutralisierte COOH-Funktion besitzen oder nicht mit der XH-Funktion reagieren;
- den Thiazinderivaten, die eine neutralisierte COOH-Funktion besitzen oder nicht mit der XH-Funktion reagieren;
- den Xanthenderivaten, die eine neutralisierte COOH-Funktion besitzen oder nicht mit der XH-Funktion reagieren;
- den Fluoronderivaten, die eine neutralisierte COOH-Funktion besitzen oder nicht mit der XH-Funktion reagieren.

**4.** Verwendung nach Anspruch 1 oder 2, wobei die farbige Verbindung aus den folgenden Gruppen ausgewählt ist:

- den Azobenzolderivaten, die eine neutralisierte COOH-Funktion besitzen oder nicht mit der XH-Funktion reagieren;
- den Xanthenderivaten, die eine neutralisierte COOH-Funktion besitzen oder nicht mit der XH-Funktion reagieren.

**5.** Verwendung nach Anspruch 1 oder 2, wobei die farbige Verbindung aus den folgenden Gruppen ausgewählt ist:

- den Azobenzolderivaten, die eine neutralisierte COOH-Funktion besitzen oder nicht mit der XH-Funktion reagieren;
- den Rhodaminderivaten, die eine neutralisierte COOH-Funktion besitzen oder nicht mit der XH-Funktion reagieren.

**6.** Verwendung nach Anspruch 1 oder 2, wobei die farbige Verbindung aus den folgenden Gruppen ausgewählt ist:

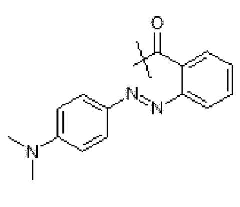
,
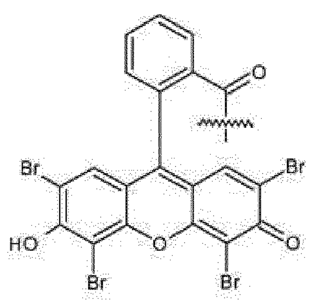

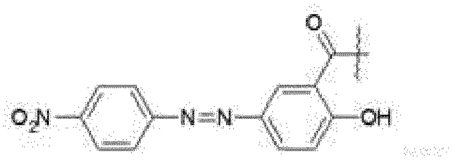

oder

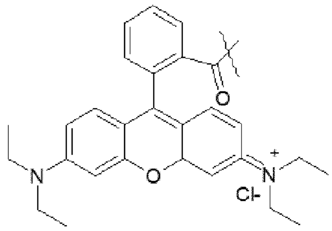

**7.** Verwendung nach Anspruch 1 oder 2, wobei die farbige Verbindung aus den folgenden Gruppen ausgewählt ist:

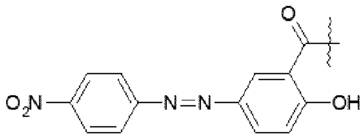

;

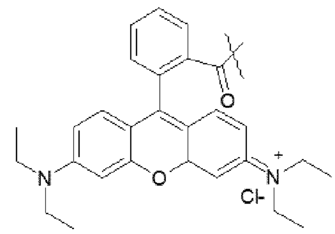

;

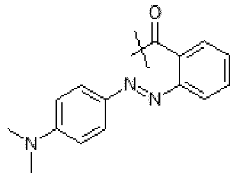

**8.** Verwendung nach einem der Ansprüche 1 bis 7, wobei die Verbindung der Formel (I) in einer Zusammensetzung (C) vorliegt, die ferner einen pH-Puffer umfasst.

**9.** Verfahren zur Bestimmung der Tonmenge, die das Fließmittel in einem Sand interkaliert, umfassend die folgenden Schritte:

a) Bereitstellen einer Verbindung der Formel (I) nach einem der Ansprüche 1 bis 7;
b) Entnehmen einer Probe des zu analysierenden Sandes;
c) Mischen der Verbindung der Formel (I) mit der Sandprobe in einem Behälter und Schütteln;
d) Filtern der in Schritt c) erhaltenen Mischung;
e) Bestimmen, in Abhängigkeit von der Farbe der in Schritt d) erhaltenen Lösung, der Tonkonzentration, die das Fließmittel im Sand interkaliert.

**10.** Verfahren nach Anspruch 9, wobei Schritt e) durch eine visuelle Bestimmung der Farbe und der Korrelation dieser Farbe mit einem Tonmengenbereich, der die Fließmittel interkaliert, oder durch photometrische Messung erfolgt, die

dann die Schritte e1) der photochemischen Messung der Adsorption des in Schritt d) erhaltenen Filtrats und einen Schritt e2) der Subtraktion des in Schritt e) erhaltenen Wertes von der photochemischen Messung der Absorption der Verbindung der Formel (I) und der Übertragung des Wertes auf eine Kalibrierungskurve aufweist, um den Ton-Gewichtsprozentsatz zu bestimmen, der die Fließmittel im Sand interkaliert.

**11.** Verfahren zur Bestimmung der Menge an Ton-Aktivitätsmodifikator-Mittel AMAA, die einem Sand zu seiner Verwendung in einer hydraulischen Bindemittelzusammensetzung zuzusetzen ist, umfassend die folgenden Schritte:

i) Bereitstellen einer Verbindung der Formel (I) nach einem der Ansprüche 1 bis 7;
ii) Entnehmen einer Probe des zu analysierenden Sandes;
iii) Mischen der Verbindung der Formel (I) mit der Sandprobe in einem Behälter und Schütteln;
iv) Filtern der in Schritt iii) erhaltenen Mischung;
v) Bestimmen der hinzuzufügenden AMAA-Menge in Abhängigkeit von der Farbe der in Schritt iv) erhaltenen Lösung.

**12.** Set für die Durchführung des Verfahrens nach den Ansprüchen 9 bis 11, umfassend:

- einen Behälter mit einem Verschluss, der eine Verbindung der Formel (I) nach einem der Ansprüche 1 bis 7 umfasst;
- einen Behälter für die Dosierung der Sandprobe;
- ein Mittel zur Entnahme von Flüssigkeit;
- ein Filtermittel;
- einen Behälter zum Auffangen des Filtrats;
- eine Kalibrierungskurve, die es ermöglicht, die Farbe mit der Tonkonzentration in Beziehung zu setzen, die das Fließmittel im Sand interkaliert, und/oder eine Korrelationskurve, die es ermöglicht, die Farbe mit der Menge des zu verwendenden Ton-Aktivitätsmodifikator-Mittels AMAA in Beziehung zu setzen.

**13.** Set nach Anspruch 12, das ferner ein Gerät zur photometrischen Messung der Adsorption umfasst.

## Claims

**1.** Use of a compound of formula (I) to determine the quantity of superplasticizer-intercalating clay in a sand and/or to determine the amount of Clay Mitigation Agent CMA to be added to a hydraulic binder composition using a sand

$$R^1\text{-}(OA)_n\text{-}XR^2 \qquad (I)$$

wherein:

$R^1$ represents a linear or branched C1 to C4 alkyl group, or a coloured compound;
$R^2$ represents a coloured compound;
A, each the same or different, independently represents a-$CH_2$-$CH_2$- group or -$CH(CH_3)$-$CH_2$- group;
n represents an integer of between 1 and 500, preferably of between 4 and 250 X is O or NH.

**2.** The use according to claim 1, wherein:

$R^1$ represents a methyl, propyl, ethyl or butyl group; and/or
A represents a -$CH_2$-$CH_2$-, -$CH(CH_3)$-$CH_2$- group; and/or
X is O or NH, preferably O; and/or
n represents an integer of between 1 and 500, preferably between 4 and 250.

**3.** The use according to claim 1 or 2, wherein the coloured compound is selected from among the following groups:

- Derivatives of azobenzene having a COOH function whether or not neutralised reacting with the XH function;
- Derivatives of acridine having a COOH function whether or not neutralised reacting with the XH function;
- Derivatives of anthraquinone having a COOH function whether or not neutralised reacting with the XH function;
- Derivatives of phthalocyanines having a COOH function whether or not neutralised reacting with the XH function;

- Derivatives of quinone having a COOH function whether or not neutralised reacting with the XH function;
- Derivatives of indophenol having a COOH function whether or not neutralised reacting with the XH function;
- Derivatives of oxazone having a COOH function whether or not neutralised reacting with the XH function;
- Derivatives of thiazine having a COOH function whether or not neutralised reacting with the XH function;
- Derivatives of xanthene having a COOH function whether or not neutralised reacting with the XH function;
- Derivatives of fluorone having a COOH function whether or not neutralised reacting with the XH function.

**4.** The use according to claim 1 or 2, wherein the coloured compound is selected from among the following groups:

- Derivatives of azobenzene having a COOH function whether or not neutralised reacting with the XH function;
- Derivatives of xanthene having a COOH function whether or not neutralised reacting with the XH function.

**5.** The use according to claim 1 or 2, wherein the coloured compound is selected from among the following groups:

- Derivatives of azobenzene having a COOH function whether or not neutralised reacting with the XH function;
- Derivatives of rhodamine having a COOH function whether or not neutralised reacting with the XH function.

**6.** The use according to claim 1 or 2, wherein the coloured compound is selected from among the following groups:

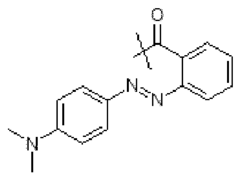

,

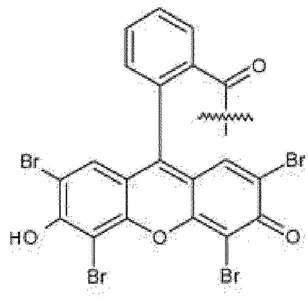

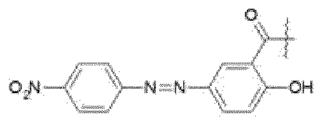

or

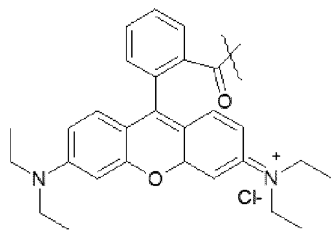

**7.** The use according to claim 1 or 2, wherein the coloured compound is selected from among the following groups:

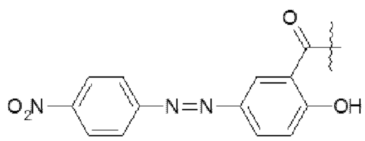

;

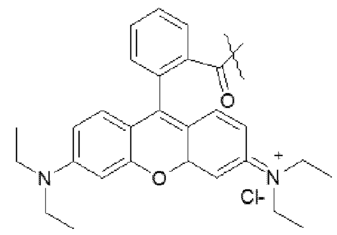

;

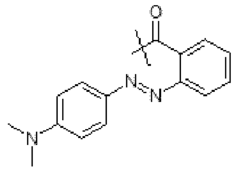

8. The use according to any one of claims 1 to 7, wherein the compound of formula (I) is in a composition (C) further comprising a pH buffer.

9. A method for determining the quantity of superplasticizer-intercalating clay in a sand, comprising the following steps:

a) Providing a compound of formula (I) according to any one of claims 1 to 7;
b) Taking a sample of the sand to be analysed;
c) Mixing the compound of formula (I) with the sand sample in a container and agitating;
d) Filtering the mixture obtained at step c);
e) Determining the concentration of superplasticizer-intercalating clays in the sand as a function of the colour of the solution obtained at step d).

10. The method according to claim 9, wherein step e) is performed by visual determination of the colour and correlation of this colour with a range of quantities of superplasticizer-intercalating clays or by photometric measurement then comprising steps e1) for photometric measurement of the absorbance of the filtrate obtained at step d) and step e2) to subtract the value obtained at step e) from the photometric measurement of the absorbance of compound of formula (I) and entering the value into a calibration curve to determine the weight percentage of superplasticizer-intercalating clays in the sand.

11. A method for determining the amount of Clay Mitigation Agent CMA to be added to a sand for use thereof in a hydraulic binder composition comprising the following steps:

i) Providing a compound of formula (I) according to any one of claims 1 to 7;
ii) Taking a sample of sand to be analysed;
iii) Mixing the compound of formula (I) with the sand sample in a container and agitating;
iv) Filtering the mixture obtained at step iii);
v) Determining the amount of CMA to be added as a function of the colour of the solution obtained at step iv).

12. A kit to implement the method according to claims 9 to 11, comprising:

- A container fitted with a stopper comprising a compound of formula (I) according to any one of claims 1 to 7;
- A container to dose the sand sample;
- A means for sampling a liquid;
- A filtering means;
- A container to recover the filtrate;
- A calibration curve allowing colour to be related to a concentration of superplasticizer-intercalating clay in the sand and/or a correlation curve allowing colour to be related to the amount of Clay Mitigation Agent CMA to be used.

13. The kit according to claim 12 further comprising an instrument for photometric measurement of absorbance.

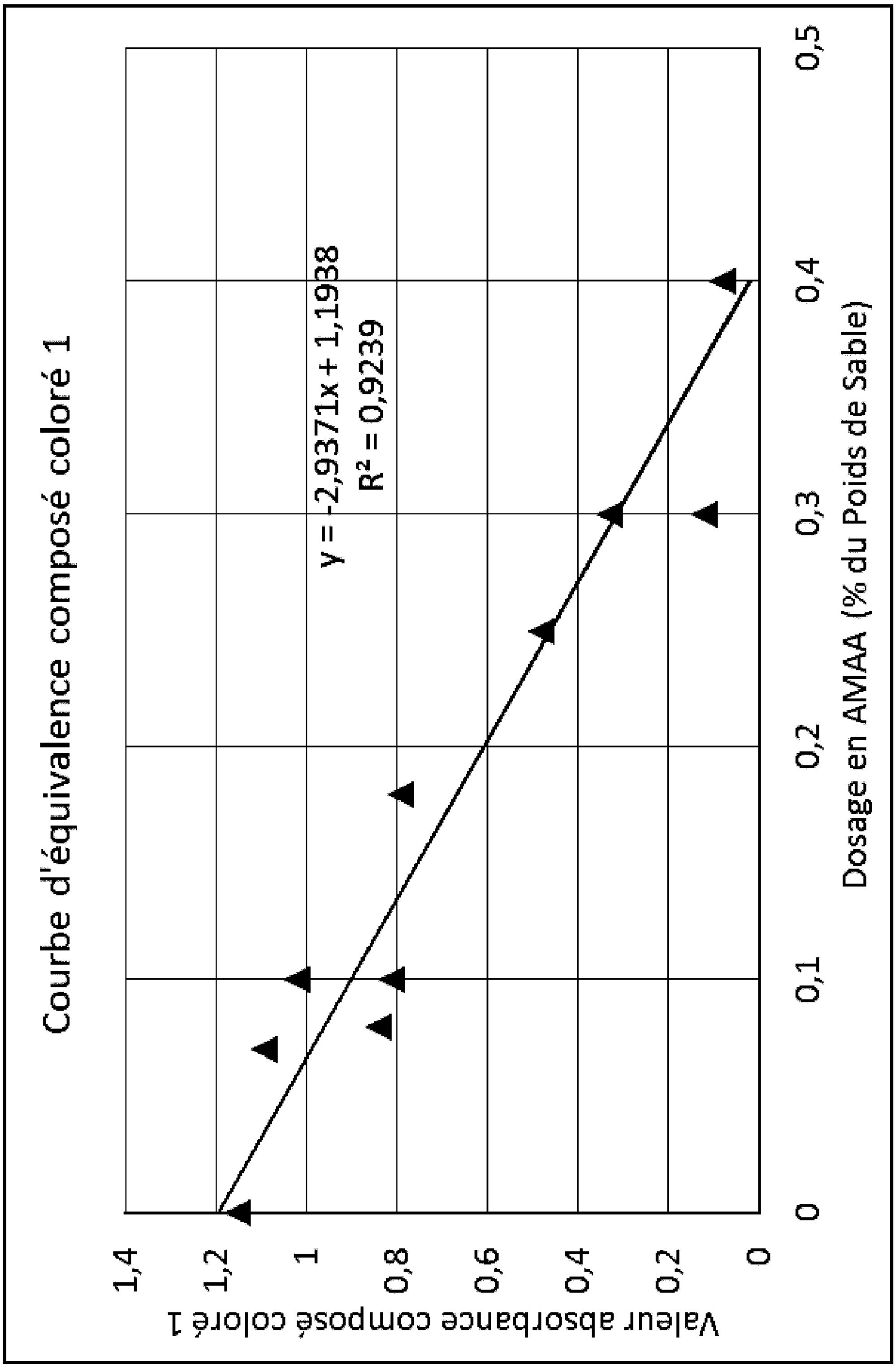
Courbe d'équivalence composé coloré 1
y = -2,9371x + 1,1938
R² = 0,9239
Valeur absorbance composé coloré 1
1,4
1,2
1
0,8
0,6
0,4
0,2
0
0
0,1
0,2
0,3
0,4
0,5
Dosage en AMAA (% du Poids de Sable)

FIG.1

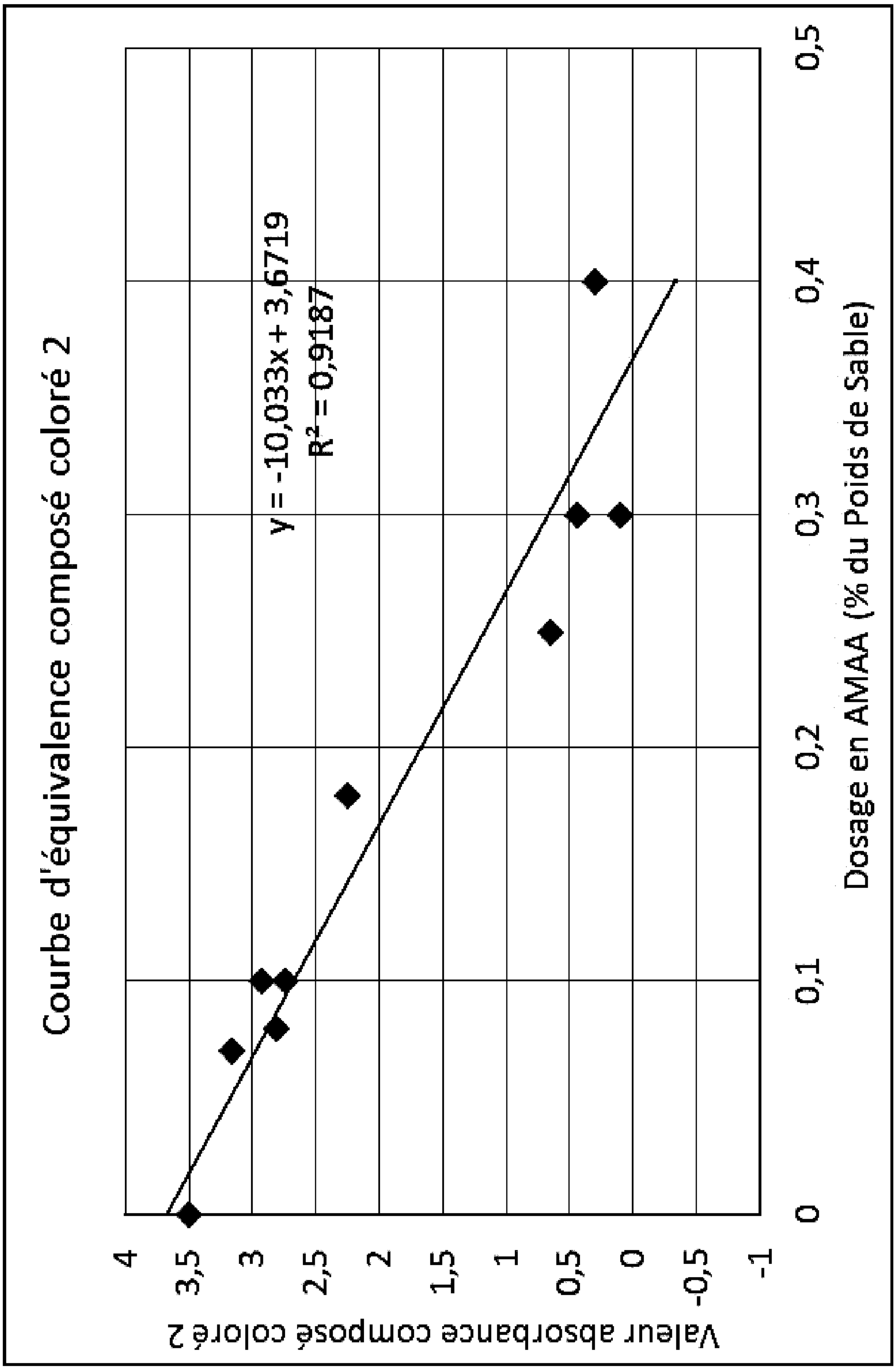
Courbe d'équivalence composé coloré 2
y = -10,033x + 3,6719
$R^2$ = 0,9187
Valeur absorbance composé coloré 2
4
3,5
3
2,5
2
1,5
1
0,5
0
-0,5
-1
0
0,1
0,2
0,3
0,4
0,5
Dosage en AMAA (% du Poids de Sable)

FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1015398 A
- WO 2011121230 A
- WO 2006032786 A
- FR 2875496
- JP H101628 A


### Littérature non-brevet citée dans la description


- **A. K. PRAJAPATI et al.** Azomesogens with methoxyethyl tail : Synthesis and characterization. *PROCEEDINGS IF THE INDIAN ACADEMY OF SCIENCES. CHEMICAL SCIENCES*, May 2005, vol. 117 (3), 255-261
- **A. K. PRAJAPATI et al.** Azomesogens containing an ethoxyethyl terminal chain : synthesis and characterization. *LIQUID CRYSTALS, TAYLOR & FRANCIS, GB*, June 2004, vol. 31 (6), 889-894
- **SHUHUA PENG et al.** Azobenzene moiety variation directing self-assembly and photoresponsive behavior of azo-surfactants. *JOURNAL OF MATERIALS CHEMISTRY C*, 21 May 2014, vol. 2 (39), 8303-8312